Europäisches Patentamt

European Patent Office

Office européen des brevets

(11) Publication number: **0 415 693 A1**

## EUROPEAN PATENT APPLICATION

(21) Application number: **90309368.0**

(22) Date of filing: **28.08.90**

(51) Int. Cl.⁵: **A61K 37/02**, //(A61K37/02, 31:485)

(30) Priority: **28.08.89 US 399590**

(43) Date of publication of application:
**06.03.91 Bulletin 91/10**

(84) Designated Contracting States:
**AT BE CH DE DK ES FR GB GR IT LI LU NL SE**

(71) Applicant: **ARIZONA TECHNOLOGY DEVELOPMENT CORPORATION**
**1430 East Fort Lowell Road, Suite 200**
**Tucson, Arizona 85719-2366(US)**

(72) Inventor: **Porreca, Frank c/o The University of Arizona**
**Department of Pharmacology, Health Sciences Center**
**Tucson, Arizona 85724(US)**

(74) Representative: **Bassett, Richard Simon et al**
**ERIC POTTER & CLARKSON St. Mary's Court**
**St. Mary's Gate**
**Nottingham NG1 1LE(GB)**

(54) Composition and method for selective enhancement of opiate activity and reduction of opiate tolerance and dependence.

(57) A composition for selectively enhancing opiate activity, including analgesic, anti-tussive, and sedative activity, as well as opiate activity in the treatment of dyspena and modulation of intestinal motility, while reducing tolerance and dependence associated with chronic use of opiate analgesics. More specifically, a composition and method which selectively enhances opiate analgesia induced at mu ($\mu$) receptors by direct or indirect action at the delta ($\delta$) receptor sites of the central nervous system.

EP 0 415 693 A1

# COMPOSITION METHOD FOR SELECTIVE ENHANCEMENT OF OPIATE ACTIVITY AND REDUCTION OF OPIATE TOLERANCE AND DEPENDENCE

## Background of the Invention

The present invention relates generally to a composition for selectively enhancing opiate activity, including analgesic, antitussive, and sedative activity, as well as opiate activity in the treatment of dyspena and modulation of intestinal motility, while concurrently reducing the rate of development of tolerance add degree of physical and psychological dependence associated with chronic use of these narcotic analgesics. More specifically, the present invention relates to a composition and method which selectively enhances opiate activity induced at mu ($\mu$) receptors by direct or indirect action at the delta ($\delta$) receptor sites to the the central nervous system. With specific regard to the analgesic activity, opiate analgesics typically act by modulating the perception of pain or nociception. It is generally accepted that all type of painful experiences, whether produced experimentally or occurring clinically, include both the crigira sensation and the reaction to that sensation. The opioid analgesics, such as morphine, hydromorphone. oxymorphone, codeine, levorphanol, methadone, tramadol and meperidine, are known to be effective in relieving clinical pain and to increase the pain threshold by increasing the capacity to tolerate experimentally induced pain. With the opioid analgesics, the sensation of pain is altered and the ability to tolerate pain is markedly increased. Thus, the opioid analgesics are known to exhibit antinociception.

Further, certain opioid analgesics exhibit known activity in the gastrointestinal tract to modulate intestinal motility or exhibit anti-tussive activity. For example, morphine is known to diminish peristalsis in the colon, relieve certain forms of dyspnea. e.g., dyspnea of acute left ventricular failure and pulmonary edema. Orally administered codeine produces a well documented antitussive effect.

All opioid drugs, however, are characterized by a significant disadvantage which substantially limits their clinical use and increases the overall likelihood for compulsive abuse. The development of tolerance and physical dependence with repeated use is characteristic of all opioid analgesics. Once physical dependence occurs, withdrawal symptoms can be severe.

Studies of the binding of opioid drugs and peptides to specific sites in the brain and other organs have suggested the existence of a multitude of opioid receptor sites. These studies have provided good evidence of four major categories of opioid receptors in the central nervous system (CNS) designated $\mu$ (mu) $x$ (kappa), $\delta$ (delta), and o (sigma). The $\mu$ receptor has been implicated in mediation of supraspinal analgesia, respiratory depression, euphoria and physical dependence; the $x$ receptor, spinal analgesia, miosis, and sedation; the o receptor, dysphoria, hallucinations, and respiratory and vasomotor stimulation. Morphine and morphine-like opiate drugs are known $\mu$ agonists. $\mu$ receptors exist on the terminal axons of the primary afferents within laminae I and II (the substantia gelatinosa) of the spinal cord and in the spinal nucleus of the trigeminal nerve. Morphine-like drugs acting at these receptor sites are thought to decrease the release of neurotransmitters, such as substance P, which mediate transmission of pain impulses. Since morphine is inactive at receptor sites at which metenkephalin, a $\delta$ agonist, inhibits neuronal firing, it is believed that separate $\mu$ and $\delta$ receptor sites participate in inhibiting transmission of pain impulses in the spinal cord. $\delta$ receptor involvement in CNS analgesia has only recently been explored. (Gilman, A.G., et al, Ed., *Goodman and Gilman's The Pharmacological Basis of Therapeutics*, 7th ed, 1985, pp. 491-499).

The role of $\mu$ and $\delta$ receptors in the modulation of supraspinal and spinal analgesia has been explored. (Porreca. F., Heyman, J., Mosberg, H., Omnaas, J. and Vaught, J., *Role of Mu and Delta Receptors in the Supraspinal and spinal Analgesic Effects of [D-Pen $^2$, D-Pen $^5$] Enkephalin in the Mouse*, J. Pharmacol. Exp. Ther. 241 393-400. 1987 (hereinafter "Porreca and Heyman")) Porreca and Heyman investigated the roles of $\mu$ and $\delta$ receptors in antinociception. under acute morphine tolerance conditions. by exploring possible cross-tolerance between morphine and a variety of receptor selective nonselective $\delta$ receptor agonists. $\delta$ receptor antagonism and the possible potentiation of $\mu$ receptor analgesia by $\delta$ agonists. Differentiation of analgesic activity at $\mu$ or $\delta$ receptor sites involved the determination of possible cross-tolerance between receptor selective agonists and morphine after induction of acute morphine tolerance. The study employed the highly selective $\delta$ agonists (D-Pen$^2$, D-Pen$^5$] Enkephalin] (DPDPE) and [D-Pen$^2$, L-Pen$^5$] Enkephalin (DPLPE), and the relatively nonselective $\delta$ ngonists [D-Ala$^2$, D-Leu$^5$] Enkephalin (DADLE), [D-Ser$^2$, Leu$^5$, Thr$^6$] Enkephalin (DSLET) and [D-Thr$^2$, Leu, Thr$^6$] Enkephalin (DTLET). h second approach determined the possible potentiation of morphine analgesia by threshold analgesic doses of the DPDPE, DPLPE, Leuenkephalin and Met-enkephalin, both endogenous $\delta$ agonists. Finally, a direct test of $\delta$ receptor involve-

ment was made using ICI 174,864, a highly selective δ antagonist.

Porreca and Heyman determined that pretreatment with a single subcutaneous (s.c.) dose of morphine displaced the intracerebroventricular (i.c.v.) morphine dose-response line 7-fold to the right, indicating acute tolerance. Morphine pretreatment, however, failed to displace the i.c.v. DPDPE or DPLPE dose-response lines indicating a lack of i.c.v. cross-tolerance between these agonists. The pre-treating dose of morphine resulted in cross-tolerance to the i.c.v. analgesic effects of [D-Ala$^2$, MePhe$^4$, Gly-ol] Enkephalin (DAGO) and [D-Ala$^2$, Met$^5$] Enkephalin (DAME), both $\mu$ agonists, DSLET and DTLET but not DPDPE. In the antagonism study, ICI 174,864 antagonized i.c.v. DPDPE analgesia without affecting i.c.v. morphine analgesia.

The findings of a lack of i.c.v. cross-tolerance between DPDPE and morphine are consistent with the observation of selective and dose related antagonism of i.c.v. DPDPE, but not i.c.v. morphine analgesia by ICI 174,864. These results suggest that the analgesic activity of δ agonists, such as DPDPE, occurs at receptor sites other than the $\mu$ receptors.

It has been reported that doses of δ agonists, such as Leu-enkephalin, which do not produce significant analgesia when given alone, increase the analgesic potency of morphine in mice under acute tolerance conditions. (Vaught, J.L. and Takemori, A.E., Differential effects of leucine and methionine enkephalin on morphine-induced analgesia, acute tolerance and dependence, J. Pharmacol. Exp. Ther., 208: 86-90, 1979). Vaught and Takemori reported that i.c.v. administration of Leu-enkephalin before or after s.c. morphine administration resulted in a marked leftward shift of the morphine dose-response curve, indicating an increase in potency. Moreover, Vaught and Takemori have indicated than administration of Leu-enkephalin, a δ agonist, also increased the rate of development of acute tolerance and dependence produced by a single dose of morphine. While at the time Vaught and Takemori was published, the involvement of specific receptor sites was less clear, it is clear that Vaught and Takemori teaches that the administration of a δ agonist potentiates the potency of a single dose of morphine and, also potentiates the development tolerance and physical dependence.

In the Porreca and Heyman study, neither i.c.v. DPDPE nor i.c.v. DADLE produced significant analgesia when given alone in the doses tested. When co-administered i.c.v. with morphine, DPDPE and DADLE significantly potentiated morphine analgesia in mice with acute morphine tolerance (single dose morphine). Potentiation is evidenced by a strong leftward shift of the dose-response curves for i.c.v. morphine alone and for co-administered i.c.v. DPDPE and DADLE. This finding is consistent with Vaught and Takemori. However, in contrast, intrathecal (i.t.) doses of DADLE and DPDPE, not producing analgesia when given alone, failed to potentiate i.t. doses of morphine. This finding supports the conclusion reached by Porreca and Heyman that at the spinal level, DPDPE does not potentiate the analgesic effects of morphine.

The specific role of the δ receptor in supraspinal and spinal antinociception has been investigated. (Heyman, J.S., Mulvaney, S.A., Mosberg, H.I., Porreca, F., Opioid δ-receptor involvement in supraspinal and spinal antinociception in mice, Brain Res., 420: 100-108, 1987 (hereinafter "Heyman and Mulvaney"). In Heyman and Mulvaney highly selective agonists and antagonists were used to demonstrate differential antagonism of the antinociceptive effects of the selective agonists in the brain and spinal cord. By blocking the supraspinal and spinal antinociceptive effects of DPDPE, but not DAGO or morphine, with ICI 174,864, supraspinal and spinal δ-opioid receptors have been implicated in antinociception. Antinociceptive dose-response curves were constructed for DAGO (a $\mu$ agonist) and for DPDPE (a δ agonist) in the absence and presence of the $\mu$-selective antagonist $\beta$-funaltrexamine ($\beta$-FNA) and of ICI 174,864 (a δ antagonist). The effective antagonism of DPDPE but not morphine or DAGO antinociception by ICI 174,864, together with the effectiveness of $\beta$-FNA against morphine and DAGO, but not DPDPE antinociception, demonstrates the involvement of both supraspinal and spinal δ receptors in mediation of antinociception.

Thus, it is known in the art that δ receptors are involved in mediation and/or modulation of the antinociceptive action of opioid drugs at the $\mu$ receptor sites in the brain and spine. As evidenced by both Porreca and Heyman and Vaught and Takemori, administration of a selective δ agonist, such as DPDPE or Leuenkephalin, increases the potency of morphine and also potentiates the undesired properties of acute tolerance and physical dependence. Each of Vaught and Takemori, Porreca and Heyman and Heyman and Mulvaney, however, address acute morphine tolerance conditions where a single pre-treating dose of morphine is administered with subsequent testing of the effect of a δ agonist on analgesia. None of the studies relevant to the interaction between the δ receptor and the $\mu$ receptor have addressed chronic morphine tolerance conditions. However, based upon the findings of Vaught and Takemori, one skilled in the art would be led to conclude that administration of a δ agonist, such as DPDPE, DPLPE or Leu-enkephalin, with an $\mu$ agonist opioid, such as morphine or codeine, would potentiate both the potency of morphine and the tolerance and physical dependence, and the withdrawal signs associated with opioid physical dependence.

In accordance with the present invention, therefore, there is provided a composition comprising a combination of an endogenous or exogenous direct or indirect δ agonist with an opiate analgesic, such as morphine, codeine or any other morphine-like drug, which when repeatedly administered (i.e. more than once), enhances potency and efficacy of the opioid drug without loss of duration of analgesia. An important aspect of the present invention is decreased development of tolerance and physical dependence when compared to equianalgesic doses of the opiate drug alone. Furthermore, the combination of enkephalinase inhibitors with morphine, as well, it is believed, as the combination of compounds which directly or indirectly result in δ receptor activation and compounds which directly or indirectly results in activation of the μ receptors, exhibit the above-described actions. The present invention exhibits the above actions when administered by any combination of peripheral or central administrative routes.

Those skilled in the art will understand and appreciate that the combination is useful and beneficial in the treatment of conditions of moderate to severe pain; any condition known to be modulated by opioid activity, such as analgesic, anti-tussive, and sedative activity, as well as opiate activity in the treatment of dyspena and modulation of intestinal motility; any condition of developed opiate tolerance and any other condition not adequately treated by opiates; any condition of developed opiate tolerance and any other condition not adequately treated by opiates.

These and other features and advantage of the present invention will become more apparent from the following more detailed description of the preferred embodiments of the invention with reference to the accompanying figures.

Brief Description of the Figures

Figure 1 represents dose-response curves for *i.c.v.* administration of morphine under increasing nociceptive stimuli.

Figure 2 represents dose-response curves for single dose *i.c.v.* administration of morphine alone and concurrently the δ agonist DPDPE.

Figure 3 represents dose-response curves for *i.c.v.* administration of morphine and morphine co-adminstered with DPDPE under chronic morphine tolerance conditions.

Figure 4 represents the time-response curves for both *i.c.v.* morphine and an equianalgesic combination of *i.c.v.* morphine plus DPDPE.

Figure 5 represents the dose-response curves under chronic morphine tolerance conditions for saline, high dose morphine, low dose morphine and morphine with DPDPE.

Figure 6 represents an evaluation of naloxone-precipitated physical dependence jumping under chronic tolerance conditions.

Figure 7 represents the dose-response curves for morphine alone and with DPDPE administered through different administrative routes.

Figure 8 represents and evaluation of naloxone-precipitated physical dependence jumping under chronic morphine tolerance induced through different administrative routes.

Figure 9 represents the dose-response curves, under acute conditions, for morphine alone and in combination with graded doses of the endogenous δ agonist Leu-enkephalin.

Figure 10 represents the dose-response curves, under chronic conditions, of saline, low dose morphine, high dose morphine and an equianalgesic combination of low dose morphine and Leu-enkephalin.

Figure 11 represents an evaluation of naloxone-precipitated withdrawal jumping, under chronic conditions, with low dose morphine, high dose morphine or an equianalgesic combination of low dose morphine and Leu-enkephalin.

Figure 12 represents the dose-response curves for morphine alone and morphine with thiorphan under increasing nociceptive stimuli.

Figure 13 represents the dose-response curves, under chronic conditions, for codeine alone, codeine and DPDPE and a combination of codeine and thiorphan.

Detailed Description of the Preferred Embodiments

In accordance with the preferred embodiments of the present invention, there is provided a composition comprising a combination of an endogenous or exogenous, direct or indirect δ agonist with an opiate analgesic, for example, without limitation, morphine, codeine or any other morphine-like drug. The following test examples are provided to illustrate the utility and operation of the preferred embodiments and are not Intended to, nor should they be construed, as limiting the scope and content of the invention. Male ICR mice (20-25 g) (Charles River Breeding Laboratories, Inc., Wilmington, MA) were used in all tests. The animals were housed in groups of eight in a temperature-controlled room. A standard 12 hr. light-dark cycle was used, with lights on at 7:00 am.

Each test employed the tail-flick assay, described in Porreca and Heyman. The tail flick assay involves a rapid flick of the test animal's tail away from heat source. The nociceptive stimulus was heated water. Graded dose-response curves were established by expressing the data according to the following formula:

% Analgesia - 100 X (test latency - control latency)/(15-control latency) where 15 represents the cutoff time (in seconds) used in order to avoid damage to the tail. Animals not withdrawing their tails within the 15 second time period cutoff were assigned a maximum analgesic score.

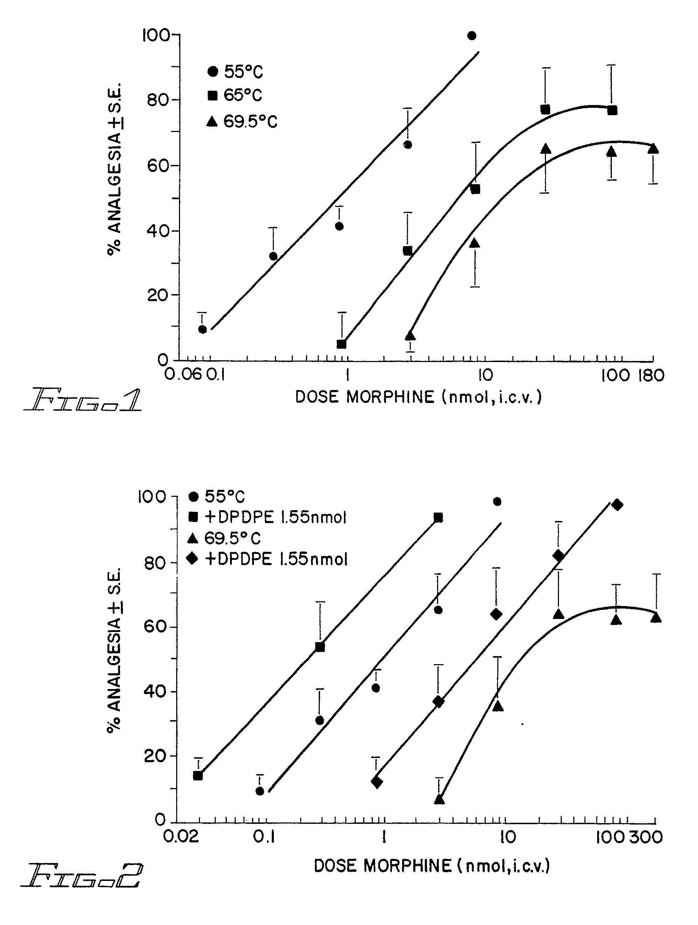

With reference to Figure 1, single *i.c.v.* dosages of morphine were given and the water tall flick test performed at water temperatures of 55° C, 65° C and at 69.5° C. As the intensity of the nociceptive stimulus (heated water) increases, the dose-response line for morphine is progressively displaced to the right and the maximum analgesic effect is reduced. Thus, the decreased potency and the decreased efficacy of morphine under conditions of severe nociceptive stimulus reveals that morphine is a partial agonist

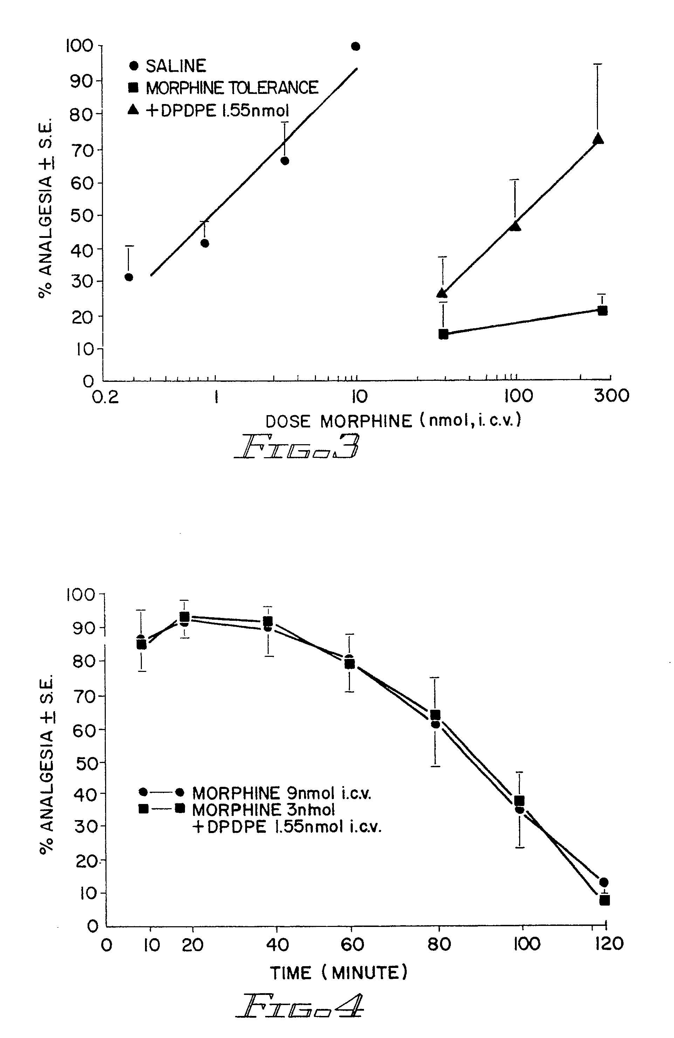

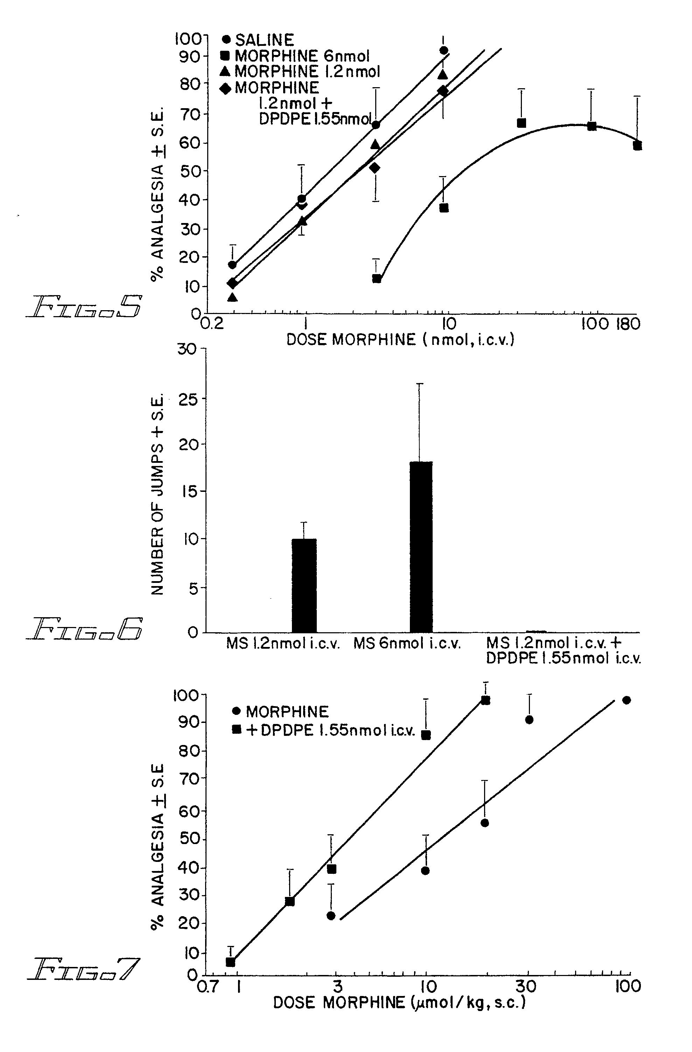

Figure 2 illustrates the dose response curves for single *i.c.v.* dosages of morphine alone or co-administered with a sub-antinociceptive dose of DPDPE, at water temperatures of 55° and 69.5° C. At 55° C, DPDPE increases the potency of morphine as evidenced by the leftward shift of the dose-response curve. At 69.5° C, DPDPE increased both potency and efficacy of morphine as evidenced by both the leftward shift and straightening of tee dose-response curve, allowing the complete (100%) production of an analgesic response at this severe nociceptive stimulus and in contrast to morphine alone under the same conditions. The test illustrated with reference to Figure 3 was conducted under chronic morphine tolerant conditions, which more closely correlates to clinical presentation of morphine to patients (i.e., several doses of the drug). Morphine or morphine plus DPDPE was administered *i.c.v.* twice daily (i.e., A.M. and P.M.) for seven days, with *s.c.* saline or *s.c.* morphine according to the following schedule: day 1, 10 mg/kg; day 2, 30 mg/kg; day 3, 100 mg/kg; day 4, 100 mg/kg; day 5, 300 mg/kg; day 6, 300 mg/kg; and day 7, 300 mg/kg. On day 8, the mice received single *i.c.v.* injections of morphine or morphine co-administered with DPDPE. The tail flick test was performed using 55° C water as the nociceptive stimulus. The mice were shown to be highly tolerant to morphine, as demonstrated by the significant rightward displacement of the morphine dose-response curve and the decreased analgesic efficacy produced by the 100 μg *i.c.v.* dosage of morphine. When co-administered with DPDPE, however, the response to *i.c.v.* morphine was increased in potency as well as a significant increase in the analgesic efficacy of morphine.

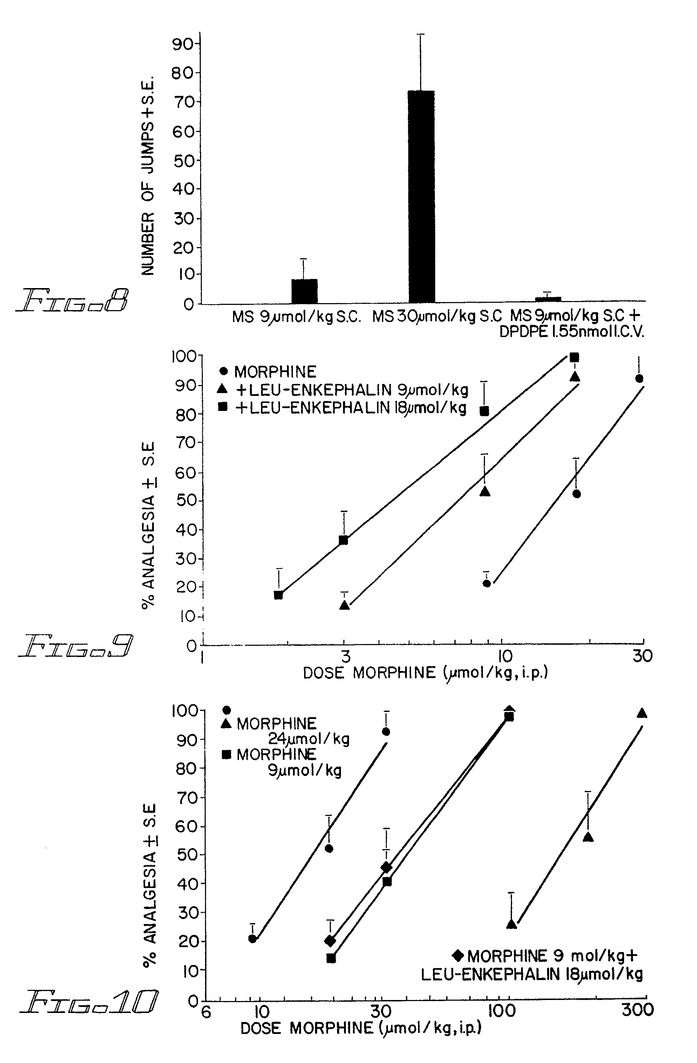

Figure 4 illustrates the time-response lines for *i.c.v.* morphine alone or an equianalgesic combination of *i.c.v.* morphine plus DPDPE in the tail flick assay. It can be seen that the combination of morphine plus DPDPE produces the same antinociceptive duration of action.

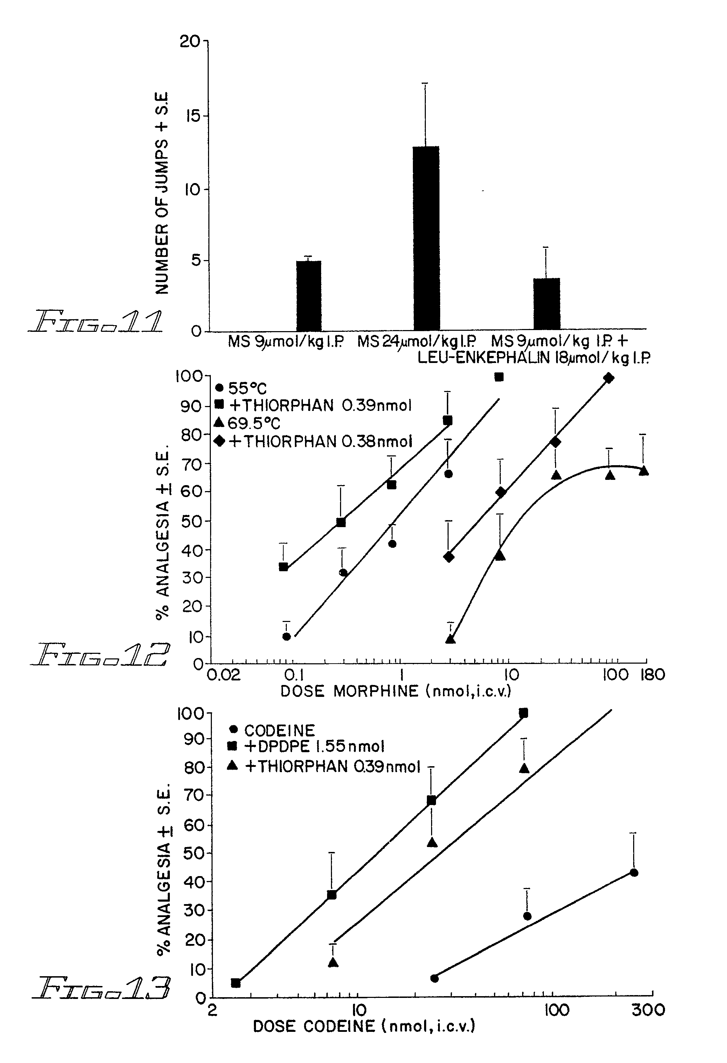

Figure 5 depicts the dose-response lines for *i.c.v.* morphine under chronic morphine tolerant conditions in response to saline, high dose morphine, low dose morphine or a combination of morphine and DPDPE. The mice were pre-treated twice daily *i.c.v.* for three days with saline, 6 nmol (2 μg) morphine, 1.2 nmol (0.4 μg) morphine, or a combination of 1.2 nmol morphine and 1.55 nmol (1 μg) DPDPE. The mice were tested on day 4, eighteen hours after the last injection. The combination of morphine plus DPDPE was shown to be equianalgesic with the high dose (6 nmol) morphine. The morphine dose-response curve was displaced to the right depending upon the dose of morphine used in the pretreatment. Pretreatment with both low dose (1.2 nmol) morphine and morphine plus DPDPE displaced the morphine dose-response line to the right (compared with saline) approximately 1.5 fold. In contrast, however, high dose (6 nmol) morphine pretreatment resulted in a 13.3 fold rightward displacement of the morphine dose-response curve and decreased the analgesic efficacy of morphine. Thus, the combination of morphine plus DPDPE produced the same analgesic response as high dose morphine and increased the potency and efficacy of low dose morphine, while unexpectedly decreasing the rate of development of morphine tolerance.

An evaluation of naloxone-induced physical dependence signs was performed using mice pretreated *i.c.v.* with twice daily injections of saline, DPDPE alone, low dose morphine, high dose morphine or equianalgesic combination of morphine plus DPDPE (as previously described) for a period of six days. One hour after the morning injection on day 6, the mice received a *s.c.* injection of naloxone, a known non-selective opiate antagonist, at a dosage of 30 mg/kg. The mice were evaluated for withdrawal jumping signs for thirty minutes. Mice pre-treated with DPDPE and saline exhibited no withdrawal jumping. As illustrated with reference to Figure 6, as expected, mice pre-treated with low dose morphine and high dose morphine exhibited significant withdrawal jumping. However, mice pre-treated with equianalgesic doses of morphine plus DPDPE did not show withdrawal jumping when challenged with naloxone. Thus, unexpectedly, the delta agonist failed to increase the rate of development of physical dependence to morphine following *i.c.v.* administration.

Figure 7 illustrates a test performed to determine whether potentiation of potency occurs where different routes of administration are used for administration of the opiate analgesic and the δ agonist. Mice were injected with *s.c.* morphine alone or with DPDPE *i.c.v.* Morphine was given *s.c.* twenty minutes prior to testing in the warn water (55 ° C) tail flick assay and *i.c.v.* DPDPE (1.55 nmol) was administered ten minutes prior to testing. It can be seen, from the leftward shift of the DPDPE dose response curve, that *i.c.v.* DPDPE increases the potency of *s.c.* morphine.

Figure 8 represents and evaluation of naloxone-precipitated physical dependence signs performed using mice pre-treated with twice daily injections of *s.c.* low dose morphine (9 $\mu$mol) and or *s.c.* high dose morphine (10 $\mu$mol), or with an equianalgesic combination of *s.c.* morphine plus *i.c.v.* DPDPE, for a period of six days. Two hours after the last injection on day 6, the mice received a *s.c.* injection of naloxone 30 mg/kg. The mice were evaluated for withdrawal jumping signs for thirty minutes. Mice pre-treated with DPDPE and saline exhibited no withdrawal jumping. As illustrated with reference to Figure 6, as expected, mice pre-treated with high dose morphine exhibited significant withdrawal jumping, while those pre-treated with low dose morphine showed less jumping (*i.e.,* less dependency). However, unexpectedly, mice pre-treated with equianalgesic doses of morphine plus DPDPE did not show withdrawal jumping when challenged with naloxone. Thus, DPDPE does not increase the rate of development of physical dependence to morphine even when administered through different routes of administration.

Leucine-enkephalin, an endogenous $\delta$ agonist, exhibits similar potentiation of morphine potency. Figure 9 illustrates the dose-response curves for acute (single injection) *i.p.* administration of morphine alone, and in combination with graded *i.p.* doses of Leu-enkephalin (9 and 18 $\mu$mol/kg, or 5 and 10 mg/kg, respectively). The leftward shift of both dose-response curves for the graded *i.p.* dosages of Leu-enkephalin with morphine is indicative of a dose-related increase in *i.p.* morphine potency.

Under chronic morphine tolerant conditions, as illustrated in Figure 10, equianalgesic combinations of Leu-enkephalin plus morphine develop tolerance to morphine at a rate much slower that with morphine alone. Mice were pre-treated *i.p.* twice daily with saline, low dose morphine (9 $\mu$mol), high dose morphine (24 $\mu$mol), or with an equianalgesic combination of low dose morphine plus Leu-enkephalin for a period of three days. Testing took place on the morning of day 4. Pretreatment with either low dose morphine or low dose morphine plus Leu-enkephalin resulted in a 2.3 rightward shift in the morphine dose-response curve, compared to mice pre-treated with saline. In contrast, however, pretreatment with high dose morphine resulted in a 9.1 fold rightward shift in the morphine dose-response line when compared to saline pretreatment

The effectiveness of Leu-enkephalin in decreasing development of opiate physical dependence was evaluation by naloxone-precipitated physical dependence jumping in chronic morphine tolerant mice. Mice were pretreated twice daily with *i.p.* low dose morphine, high dose morphine or an equianalgesic combination of low dose morphine plus Leu-enkephalin (as previously described) for a period of six days. Testing took place two hours after the last injection, and jumping was monitored for thirty minutes after the *s.c.* injection of naloxone (30 mg/kg). It is clear from Figure 11 that the group receiving the Leu-enkephalin plus morphine showed significantly less jumping than the group receiving high dose morphine, and also less jumping that the group receiving low dose morphine. Thus, co-administration of Leu-enkephalin does not enhance, but unexpectedly decreases, the development of opiate physical dependence.

To further test the involvement of $\delta$ activity in mediation nociception, the indirect $\delta$ agonist thiorphan, an enkephalinase inhibitor, was administered concurrently with morphine at 0.1 $\mu$g, a dose which does not produce analgesia alone. Figure 12 illustrates the dose-response lines for *i.c.v.* morphine alone and *i.c.v.* morphine co-administered with thiorphan. The tail flick assay was conducted at 55 ° C and at 69.5 ° C. At the lover temperature, thiorphan was shown to increase $\delta$ receptor activity indirectly, presumably through inhibition of breakdown of the endogenous $\delta$ agonists. At the higher temperature, thiorphan increased both the potency and efficacy of morphine. Thus, co-administration of thiorphan increased both the potency, as represented by the leftward shift of the morphine dose response curve, and efficacy, as represented by the straightening of the morphine dose-response line, of opiate $\mu$ agonists such as morphine.

Finally, to test the potentiation activity of a $\delta$ agonist on another opiate $\mu$ agonist, mice were pre-treated with codeine alone, codeine plus DPDPE (1.55 nmol) and codeine plus thiorphan (0.39 nmol). The dose-response lines for codeine administration are illustrated with reference to Figure 13. It can be readily appreciated that the mice receiving codeine along did not experience substantial pain relief, as indicated by the low maximal effect (efficacy) related to production of about 40% analgesia. However, with co-administration of DPDPE or thiorphan, substantial potentiation of both potency, the leftward shift of the dose response curve, and efficacy, the increase in maximum analgesia, were achieved.

It will be understood and appreciated, by those skilled in the art, therefore, that a new combination has been specifically disclosed and described. Specifically, by combining a $\delta$ receptor agonist, with an opiate analgesic, which are $\mu$ agonists,

both the potency and efficacy of the opiate analgesic are increased, without any increase, but a decrease, in the development of tolerance and physical dependence. Moreover, those skilled in the art will understand that the foregoing test example are for illustrative purposes and generally illustrate the utility of direct, indirect, endogenous and exogenous $\delta$ agonists in combination with opiate analgesics. Examples of the class of $\delta$ agonists contemplated by the present invention are the following: [D-Pen$^2$, D-Pen$^5$] Enkephalin] (DPDPE), [D-Pen$^2$, L-Pen$^5$] Enkephalin (DPLPE), [D-Ala$^2$, D-Leu$^5$] Enkephalin (DADLE) [D-Ser$^2$, Leu$^5$, Thr$^6$] Enkephalin (DSLET), [D-Thr$^2$, Leu, Thr$^6$] Enkephalin (DTLET), Leucine-enkephalin, thiorphan, kelatorphan, (R)-3-(N-hydroxy-carboxamido-2-benzylpropanoyl)-L-phenylanine, the orally active prodrug enkephalinase inhibitor (S)-N-[N-1-[[2,2-dimethyl-1,3-dioxolan-4yl)methoxy]carbonyl]-2-phenylethyl]-L-phenylanaline]-$\beta$-alanine (SCH 34826), and its de-esterified active constituent N-[L-(-1-carboxy-2-phenyl)ethyl]-L-phenylalanyl-$\beta$-alanine (SCH 32615). Further, it will be understood by those skilled in the art that the opioid activity of any $\mu$ agonist, whether an opiate analgesic such as morphine, hydromorphone, oxymorphine, metopon, codeine, hydrocodone, drocode, oxycodone, pholcodine, levorphanol, methadone, dipipanone, phenadoxone, tramadol, meperidine or alphaprodine, or other compound acting as a $\mu$ agonist, should be potentiated both in potency and efficacy when combined in the manner disclosed by the present invention, for use an an analgesic, anti-tussive, and sedative, as well as in the treatment of dyspena and modulation of intestinal motility.

## Claims

1. The use of a pharmaceutical composition comprising a $\delta$ receptor agonist and an opiate analgesic in the manufacture of a medicament for selective enhancement of the efficacy of opiate activity and reduction of opiate tolerance and dependence.

2. A use according to Claim 1, wherein said $\delta$ receptor agonist is a direct acting agonist.

3. A use according to Claim 1, wherein said & receptor agonist is an indirect acting agonist.

4. A use according any one of the preceding claims, wherein said $\delta$ receptor agonist is an endogenous agonist.

5. A use according to any one of Claims 1 to 3, wherein said $\delta$ receptor agonist is an exogenous agonist.

6. A use according to any one of the preceding claims, wherein said $\delta$ receptor agonist is a prodrug.

7. A use according to any one of the preceding claims, wherein said composition further comprises any pharmaceutically acceptable carrier.

8. A use according to Claim 1, wherein said $\delta$ receptor agonist is selected from the group consisting of [D-Pen$^2$, D-Pen$^5$] Enkephalin], [D-Pen$^2$, L-Pen$^5$] Enkephalin, [D-Ala$^2$, D-Leu$^5$] Enkephalin, [D-Ser$^2$, Leu$^5$, Thr$^6$] Enkephalin, [D-Thr$^2$, Leu, Thr$^6$] Enkephalin, Leucine-enkephalin, thiorphan, kelatorphan, (R)-3-(N-hydroxy-carboxamido-2-benzylpropanoyl)-L-phenylalanine, (S)-N-[N-1-[[2,2-dimethyl-1,3-dioxolan-4yl)methoxy]carbonyl]-2-phenylethyl]-L-phenylalanine]-$\beta$-alanine, and N-[L-(-1-carboxy-2-phenyl)ethyl]-L-phenylalanyl-$\beta$-alanine.

9. A use according to Claim 1 or 8, wherein said opiate analgesic is selected from the group consisting of morphine, heroin, hydromorphone, oxymorphine, metopon, codeine, hydrocodone, drocode, oxycodone, tramadol, pholcodine, levorphanol, methadone, dextromoramide, dipipanone, phenadoxone, meperidine and alphaprodine.

10. A use according to any one of the preceding claims wherein the composition is intended for administration to patients as a long term treatment or to patients dependent on or tolerant to an opiate.

11. A kit comprising a pharmaceutical formulation of a $\mu$ receptor agonist and, separate or as part of the same formulation, a pharmaceutical formulation of a delta receptor agonist and means to administer the formulation or formulations to a patient, wherein there are respective said formulations for the $\mu$ and $\delta$ receptor agonists and the kit is adapted for administration of said $\delta$ receptor agonist through a different and distinct route of administration from the route of administration of said $\mu$ receptor agonist.

12. A kit according to Claim 10, 11 or 12, wherein said kit is adapted for intracerebroventricular, subcutaneous or intraperitoneal administration of one or both formulations.

FIG.1

FIG.2

FIG-3

FIG-4

FIG.5

FIG.6

FIG.7

FIG.8

FIG.9

FIG.10

11

FIG.11

FIG.12

FIG.13

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 361 553  (H.H. LOH)<br>* Column 7, line 5 - column 8, line 28 (claims 1-11) *<br>— — — | 1-12 | A 61 K 37/02 //<br>(A 61 K 37/02<br>A 61 K 31:485) |
| A | WO-A-8 903 211  (MATRIX TECHNOLOGIES, INC.)<br>— — — — — | 1-12 | |

TECHNICAL FIELDS
SEARCHED (Int. Cl.5)

A 61 K

The present search report has been drawn up for all claims

| Place of search | Date of completion of search | Examiner |
|---|---|---|
| The Hague | · 13 November 90 | BRINKMANN C. |